# EUROPEAN PATENT APPLICATION

(11) **EP 4 186 482 A1**
(43) Date of publication of application: **31.05.2023**
(21) Application number: 21864139.7
(22) Date of filing: 20.08.2021
(51) Int. Cl.: A61F 13/47, A61F 13/49, A61F 13/514, A61F 13/56

(54) **PULL-UP DISPOSABLE DIAPER**

(30) Priority: 02.09.2020 JP 2020147795
(71) Applicant: Daio Paper Corporation, Ehime 799-0492 (JP)
(72) Inventor: MORITANI, Akie, Shikokuchuo-shi, Ehime 799-0113 (JP)
(74) Representative: Williams Powell
(86) International application number: PCT/JP2021/030566
(87) International publication number: WO 2022/050081

(57) **Abstract**

The pad-type disposable diaper allows, with the diaper attached to underwear, easy checking for appropriate positioning. The diaper has a pressure-sensitive adhesive layer on the under faces of a first fixing zone located in the crotch section and the second fixing zones located forward and backward of the first fixing zone, and is to be fixed to the underwear with at least one of the front and back end portions thereof sticking out of the waist opening of the underwear. The at least one of the front and back end portions is provided with right and left marks indicative of the front-back positions so as to be visually observable from the underside of the diaper, and the marks are spaced apart from each other in the width direction and arranged in symmetry with respect to the center line passing the center of the width of the diaper.

## Description

### FIELD OF ART

The present invention relates to pad-type disposable diapers which are fixed for use to the interior surface of underwear bottoms, such as briefs and shorts (referred to simply as underwear hereinbelow).

### BACKGROUND ART

An example of such pad-type disposable diapers may be pad-type disposable diapers to be fixed to the interior surface of underwear and used for the purpose of saving additional cost incurred by changing diapers, or toilet training (for example, see Patent Publications 1 and 2).

Pad-type disposable diapers to be fixed to underwear generally have a fixing zone provided with a pressure-sensitive adhesive layer on the under face thereof, and the under face of this fixing zone is to be adhered to the interior surface of a diaper or underwear to keep the pad from being displaced while worn.

It is common with such a pad-type disposable diaper to first align the crotch section of the diaper (usually, the middle of the diaper in the front-back direction) to the crotch section of the underwear, and fix a first fixing zone in the crotch section of the diaper to the interior surface of the underwear with the pressure-sensitive adhesive layer on the under face of the first fixing zone, and then press second fixing zones located forward and backward of the first fixing zone onto the interior surface of the underwear to fix the second fixing zones thereto with the pressure-sensitive adhesive layer on the under face of the second fixing zones. It is also common to carry out this fixing process by attaching the pad-type disposable diaper to the interior face of the underwear before the underwear is worn.

The conventional pad-type disposable diaper, when worn by such a common procedure, may pose problems that the second fixing zones tend to be fixed to the underwear at a slant with respect to the center line passing the center of the width of the underwear, and that the wearer is hard to be aware of the slant of the second fixing zones. Use of the diaper in such a slanted state may cause leakage or deterioration of wearing comfort, so that solution to this problem is demanded.

### PRIOR ART PUBLICATION

### PATENT PUBLICATION

Patent Literature 1: JP 2003-093438 A
Patent Literature 2: JP 2020-049018 A

### SUMMARY OF THE INVENTION

### PROBLEMS TO BE SOLVED BY THE INVENTION

It is a primary object of the present invention to provide a pad-type disposable diaper that allows easy and appropriate positioning and fixing to underwear.

### MEANS FOR SOLVING THE PROBLEM

The pad-type disposable diaper which achieves the above-mentioned object is as follows:

### <First Aspect>

A pad-type disposable diaper having a crotch section including a middle of the diaper in a front-back direction, a front section extending forward of the crotch section, and a back section extending backward of the crotch section,
the diaper having a first fixing zone located in the crotch section, and second fixing zones located forward of and backward of the first fixing zone,
the diaper including:
   an absorber extending over the front section, the crotch section, and the back section, and
   a pressure-sensitive adhesive layer provided on under faces of the first fixing zone and the second fixing zones,
   wherein the diaper is to be fixed to underwear with at least one of front and back end portions thereof sticking out of an edge of a waist opening of the underwear,
   wherein the at least one of the front and back end portions of the diaper is provided with a pair of right and left marks indicative of positions in the front-back direction so as to be visually observable from an underside of the diaper, and
   wherein the marks in pair are spaced apart from each other in a width direction and are arranged in symmetry with respect to a center line passing a center of a width of the diaper.

### <Effect>

In attaching the present pad-type disposable diaper to underwear, the crotch section of the diaper (usually the middle of the diaper in the front-back direction) is aligned to the crotch section of the underwear, and the first fixing zone in the crotch section of the diaper is fixed to the interior surface of the underwear with the pressure-sensitive adhesive layer on the under face of the first fixing zone, and then, by means of the marks provided in the at least one of the front and back end portions of the diaper, positioning of the corresponding at least one of the second fixing zones may be checked. Specifically, the second fixing zones are placed on the interior surface of the underwear so that at least one of the front and back end portions of the diaper sticks out of the waist opening edge of the underwear. Then, each of the at least one sticking-out portion is visually observed with respect to the underwear to adjust the positioning of the corresponding second fixing zone so that the marks in pair are observed in bilateral symmetry. In this positioning, the second fixing zone, if already adhered with its pressure-sensitive adhesive layer, is peeled off and repositioned before it is re-adhered. This repositioning may be repeated as necessary. In this way, by means of the marks provided in at least one of the end portions of the diaper, the corresponding second fixing zone may be fixed in an appropriate position on the underwear more easily.

### <Second Aspect>

The pad-type disposable diaper according to the first aspect,
wherein each of the marks in pair includes scale marks arranged at regular intervals in the front-back direction.

### <Effect>

With each of the marks in pair including scale marks arranged at regular intervals in the front-back direction, the waist opening edge of the underwear may be checked for positioning with respect to the scale marks on the respective right and left sides, so that the appearance of the marks in pair with respect to the underwear may preferably be visually checked for bilateral symmetry more easily.

### <Third Aspect>

The pad-type disposable diaper according to the first or second aspect,
wherein the diaper has a region containing the absorber, and side flaps exclusive of the absorber and extending laterally outwards beyond opposed side edges of the region containing the absorber, and
wherein the marks in pair are positioned in the end portion corresponding to respective side edge areas opposed in the width direction of the region containing the absorber.

### <Effect>

It is preferred that the marks in pair are arranged spaced apart from each other with a larger distance in the width direction. However, with a pad-type disposable diaper having side flaps, the pair of right and left marks provided on the side flaps, which are prone to deformation, may be hard to be visually checked for accurate bilateral symmetry due to, for example, the areas of the marks being flexed. On the other hand, as the region containing the absorber is relatively higher in rigidity and thus less deformable, the right and left marks in pair provided in this region, as well as forward and backward thereof, may easily be visually checked for accurate bilateral symmetry, as the areas of the marks are not vulnerable to flexing.

### <Fourth Aspect>

The pad-type disposable diaper according to any one of the first to third aspects,
wherein the at least one of the front and back end portions has on an under face thereof a pressure-sensitive adhesive layer.

### <Effect>

After the first fixing zone and the second fixing zones are fixed on the interior surface of the underwear with the at least one of the front and back end portions of the diaper sticking out of the waist opening edge of the underwear, the at least one sticking-out portion, if left as it is, may deteriorate wearing comfort or air permeability, and thus may preferably be folded back toward the crotch section. However, the at least one sticking-out portion merely folded back may return to its initial state. In view of this, with the pressure-sensitive adhesive layer according to the present aspect, the at least one sticking-out portion of the diaper sticking out of the waist opening edge of the underwear is not only folded back toward the crotch section, but this folded portion may also be fixed on the exterior surface of the underwear with the pressure-sensitive adhesive layer.

### <Fifth Aspect>

The pad-type disposable diaper according to any one of the first to fourth aspects, further including a plurality of easy-fold lines arranged at intervals in the front-back direction and each extending in the width direction, for having the at least one of the front and back end portions folded back toward the underside.

### <Effect>

After the first fixing zone and the second fixing zones are fixed on the interior surface of the underwear with the at least one of the front and back end portions of the diaper sticking out of the waist opening edge of the underwear, the at least one sticking-out portion, if left as it is, may deteriorate wearing comfort or air permeability, and thus may preferably be folded back toward the crotch section. With the easy-fold lines according to the present aspect, such folding may preferably be facilitated.

### EFFECT OF THE INVENTION

According to the present invention, pad-type disposable diapers may advantageously be provided, which may easily be fixed to the appropriate position on the underwear.

### BRIEF DESCRIPTION OF THE DRAWINGS

Fig. 1 is a plan view of a pad-type disposable diaper in its spread state, illustrating the top side thereof.
Fig. 2 is a plan view of the pad-type disposable diaper in its spread state, illustrating the underside thereof (for ready conception of the figure, the marks are omitted).
Fig. 3 is a cross-sectional view taken along lines A-A in Fig. 1.
Fig. 4 is a cross-sectional view taken along lines B-B in Fig. 1.
Fig. 5 is a sectional view taken along lines C-C in Fig. 1.
Fig. 6 is a perspective view schematically illustrating the manner of use of the pad-type disposable diaper.
Fig. 7 is a plan view of a pad-type disposable diaper in its spread state, illustrating the underside thereof.
Fig. 8 is a plan view of a pad-type disposable diaper in its spread state, illustrating the underside thereof.
Fig. 9(a) is a cross-sectional view schematically illustrating the fixed state of the first fixing zone, and Fig. 9(b) is a cross-sectional view schematically illustrating the fixed state of the second fixing zones.
Fig. 10 is a front view schematically illustrating the underwear with the pad-type disposable diaper attached thereto.
Fig. 11 is a sectional view of the folded portion.
Fig. 12 is a plan view of a pad-type disposable diaper in its spread state, illustrating the underside thereof.
Fig. 13 is a side view schematically illustrating the front/back end portion of the pad-type disposable diaper.

### EMBODIMENTS FOR CARRYING OUT THE INVENTION

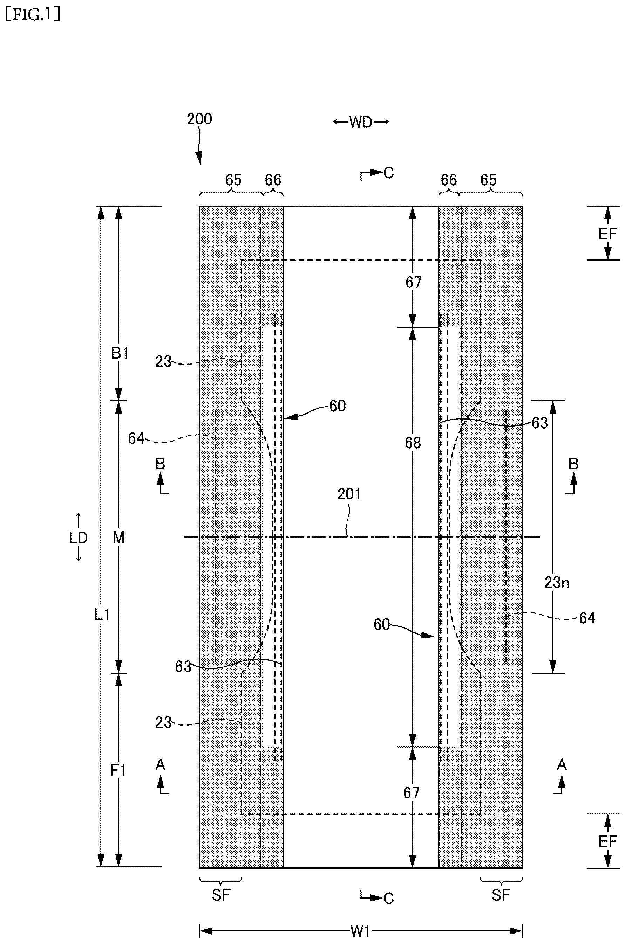

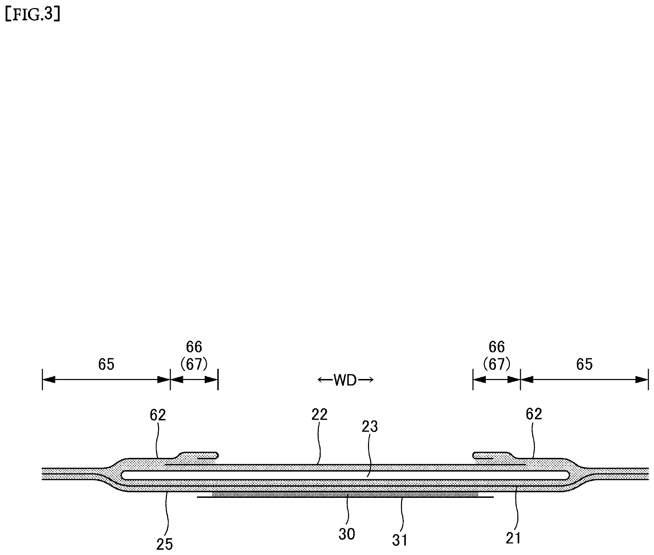

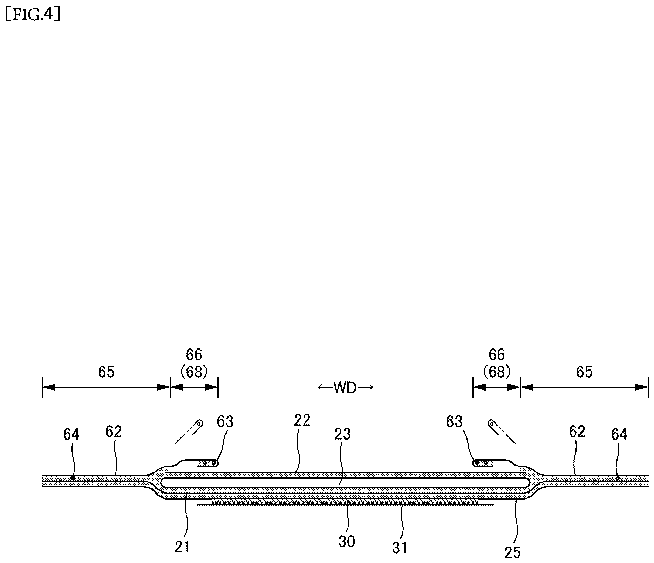

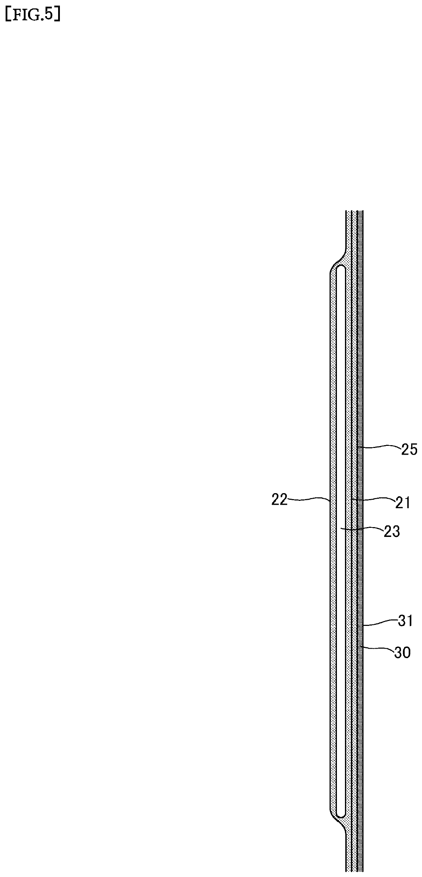

Examples of the pad-type disposable diaper will now be explained with reference to the attached drawings. Figs. 1 to 5 show a pad-type disposable diaper 200. This pad-type disposable diaper 200 has a crotch section M including the middle 201 of the diaper 200 in the front-back direction LD, a front section F1 extending forward of the crotch section M, and a back section B1 extending backward of the crotch section M. The dimensions of each section may suitably be decided and, for example, the overall length (length in the front-back direction) L1 of the product may be about 200 to 480 mm, and the overall width W1 may be about 120 to 200 mm. When absorber 23 has narrowed portion 23n as will be discussed later, the crotch section M refers to the extent in the front-back direction LD containing the narrowed portion 23n, whereas when the absorber 23 is without the narrowed portion 23n but the product has a narrowed portion formed in the middle portion thereof in the front-back direction LD with a width narrower than that of the portions forward and backward thereof (not shown, as this refers to a commonly known shape, like an hourglass shape), the crotch section M refers to the extent in the front-back direction LD containing this narrowed portion. When neither the absorber 23 nor the product contour has a narrowed portion, the crotch section M refers to the section located in the middle portion in the front-back direction LD and having a dimension in the front-back direction LD of 20 to 40% the overall length of the product.

The pad-type disposable diaper 200 has an absorber 23 contained therein and extending over the front section F1, the crotch section M, and the back section B1, a liquid pervious top sheet 22 covering the top side of the absorber 23, and a liquid-impervious sheet 21 covering the underside of the absorber 23. Materials and features of each part will now be explained in turn. Note that, as the nonwoven fabric in the description hereinbelow, commonly known nonwoven fabric may suitably be used depending on the parts or purposes. Examples of the constituent fibers of the nonwoven fabric include, but not limited to, synthetic fibers, such as polyolefin-based, e.g., polyethylene or polypropylene, polyester-based, or polyamide-based fibers (including not only single component fibers, but also composite fibers, such as of core/sheath type), as well as regenerated fibers, such as rayon or cupra, or natural fibers, such as cotton, and also mixtures thereof. For improved flexibility of the nonwoven fabric, the constituent fibers may preferably be crimped fibers. The constituent fibers of the nonwoven fabric may also be hydrophilic fibers (including those rendered hydrophilic with hydrophilizers), hydrophobic fibers, or water-repelling fibers (including those rendered water-repelling with water repellents). Further, nonwoven fabric may generally be categorized into discontinuous fiber nonwoven fabric, continuous fiber nonwoven fabric, spunbonded nonwoven fabric, melt blown nonwoven fabric, spunlace nonwoven fabric, thermal bonded (air through) nonwoven fabric, needle-punched nonwoven fabric, point-bonded nonwoven fabric, composite nonwoven fabric (SMS or SMMS nonwoven fabric having a melt blown layer interposed between spunbonded layers), or the like, generally depending on the length of the fibers, method of forming the sheet, method of joining the fibers, or layered structure, and any of these nonwoven fabric may be used.

The absorber 23 has a basic structure of an accumulated body of pulp fibers, an assembly of filaments, such as of cellulose acetate, or nonwoven fabric, to which high-absorber polymers is, e.g., mixed or fixed, as necessary. The absorber 23 may be wrapped with crepe paper or nonwoven fabric (not shown), where necessary. The absorber 23 may be in a suitable shape, such as rectangular, and in the illustrated embodiment, has a narrowed portion 23n in the crotch section M, with a width narrower than that of the portions forward and backward thereof (generally hourglass shape). The fiber basis weight of the absorber 23 and the basis weight of the absorbent polymer may suitably be decided, and the fiber basis weight may preferably be about 100 to 600 g/m², and the basis weight of the absorbent polymer may preferably be 0 to about 400 g/m².

The absorber 23 is provided on its underside with a liquid-impervious sheet 21. The liquid-impervious sheet 21 may be formed of, e.g., a polyethylene film, or a sheet having moisture permeability without impairing water shielding property for preventing dampness. Such a water-shielding, moisture-permeable sheet may be a microporous sheet obtained by kneading an inorganic filler in a polyolefin-based resin, such as polyethylene or polypropylene, in a molten state, forming the resulting mixture into a sheet, and then uni- or biaxially drawing the sheet. In the illustrated embodiment, the liquid-impervious sheet 21 extends beyond and around the absorber 23, but not necessarily.

The outer surface (under face) of the liquid-impervious sheet 21 is covered with an exterior sheet 25, which may be various kinds of nonwoven fabric.

The top side of the absorber 23 is covered with the top sheet 22. In the illustrated embodiment, the absorber 23 partially extends beyond the side edges of the top sheet 22, but the width of the top sheet 22 may be enlarged so as not to be exceeded by the side edges of the absorber 23. The top sheet 22 may be perforated or imperforated nonwoven fabric or perforated plastic sheet.

The front and back end portions of the pad-type disposable diaper 200 form end flaps EF exclusive of the absorber 23, which extend forwardly and backwardly, respectively, beyond the front and back ends, respectively, of the absorber 23. The materials constituting the end flaps EF are not particularly limited, and in the illustrated embodiment, the end flaps EF may be made up of extensions of the liquid-impervious sheet 21, the exterior sheet 25, and the top sheet 22, which extend forward and backward of the absorber 23.

The opposed lateral edge portions of the pad-type disposable diaper 200 form side flaps SF exclusive of the absorber 23, which extend laterally beyond the opposed lateral edges of the absorber. The materials constituting the side flaps SF are not particularly limited, and in the illustrated embodiment, the side flaps SF may be made up of extensions of the liquid-impervious sheet 21, the exterior sheet 25, the top sheet 22, and gathered sheet 62, which extend laterally in the opposed width directions WD of the absorber 23. Bonding of the materials, including the above, may be achieved with a hot melt adhesive, which is shown in the dotted pattern in the figures, or by material melt-bonding, such as heat sealing or ultrasonic sealing.

### <Standup Gather Part>

In order to block urine or loose stool migrating in the width direction on the top sheet 2 to thereby prevent side leakage, on the lateral sides opposed in the width direction WD of the top face of the disposable diaper are provided, all along the front-back direction, standup gather parts 60 standing up (protruding) from the opposed side portions of the top sheet 22 toward the skin.

Each of the standup gather parts 60 in the illustrated embodiment has a root zone 65 fixed to the region including the corresponding side flap SF and a protruding zone 66 extending from the root zone 65. The front and back end portions of the protruding zone 66 is fixed in a laid down state to form laid-down portions 67, and the portion of the protruding zone 66 between the front and back laid-down portions is an unfixed standup portion 68. Gathering elastic members 63 is fixed stretched in the front-back direction in at least the free edge area of the standup portion 68. Each standup gather part 60 is formed of a gathered sheet 62 folded in double to form the free edge, and a plurality of the gathering elastic members 63 may be arranged at intervals in each standup gather part as shown in the illustrated embodiment, or only one of them may be disposed in each standup gather part. The stretch rate of the area containing the gathering elastic members 63 is not particularly limited, and usually may be preferably 150 to 350%, more preferably 200 to 300%.

The root zone 65 of each standup gather part 60 in the illustrated embodiment is provided only in the corresponding side flap SF and joined to the lateral area of the liquid-impervious sheet 21 and the lateral area of the exterior sheet 25, but may extend from the side flap SF to the lateral area of the region overlapping the absorber 23.

The protruding zone 66 of each stand up gather part 60 has the laid-down portions formed in the end portions in the front-back direction LD, and the unfixed standup portion 68 formed between the end portions, and the latter standup portion 68 stands up under the contracting force of the gathering elastic members 63. The diaper, when worn, takes a vessel-like shape to fit on the body while the contracting force of the gathering elastic members 63 acts, so that the standup gather parts 60 stand up under the contracting force of the gathering elastic members 63 to be elastically brought into close contact onto the legs. As a result, so called side leakage around the legs may be kept from occurring.

The gathered sheet 62 may be a plastic sheet or melt-blown nonwoven fabric and, in view of the touch on the skin, nonwoven fabric subjected to water-repellent treatment with silicone or the like may preferably be used. The gathering elastic members 63 may be made of a material that is usually used, for example, polystyrene rubber, polyolefin rubber, polyurethane rubber, polyester rubber, polyurethane, polyethylene, polystyrene, styrene-butadiene, silicone, or polyester, and formed into, for example, a thread, string, or tape shape.

### <Side Gathers>

Each of the side flaps SF is provided, over the extent in the front-back direction LD including the crotch section M, with an elongate side elastic member 64 extending in the front-back direction LD, and the region containing the side elastic member 64 has a stretchable area (side gathers) which is contracted in the front-back direction LD due to the contraction of the side elastic member 64 and is stretchable in the front-back direction LD. The side elastic member 64 may be arranged between the gathered sheet 62 and the liquid-impervious sheet 21 in the laterally outer area in the width direction WD of the joined region of the gathered sheet 62 in the vicinity of the joining start edge as in the illustrated embodiment, or may be arranged between the liquid-impervious sheet 21 and the exterior sheet 25 in the side flap SF. Only one side elastic member 64 may be arranged on each side as in the illustrated embodiment, or a plurality of the side elastic members 64 may be arranged on each side. The side elastic member 64 may be made of the material similar to that of the gather elastic members 63.

The side elastic member 64 may be present only in each stretchable area, or may extend forward, backward, or both direction, of the stretchable area. In the case of the latter, the side elastic member 64 is finely cut at one or a plurality of locations, or is not fixed to the sheets between which the side elastic member 64 is interposed, or both, in the areas other than the stretchable area to render the areas non-stretchable (substantially equivalent to the absence of elastic members). The extent in the front-back direction LD of the stretchable area may suitably be decided, and may preferably include at least the crotch section M and, when the absorber 23 has a narrowed portion 23n, may preferably extend in both the front and back directions LD up to substantially the same front-back positions as the front and back ends of the narrowed portion 23n.

<Pressure-Sensitive Adhesive Layer>

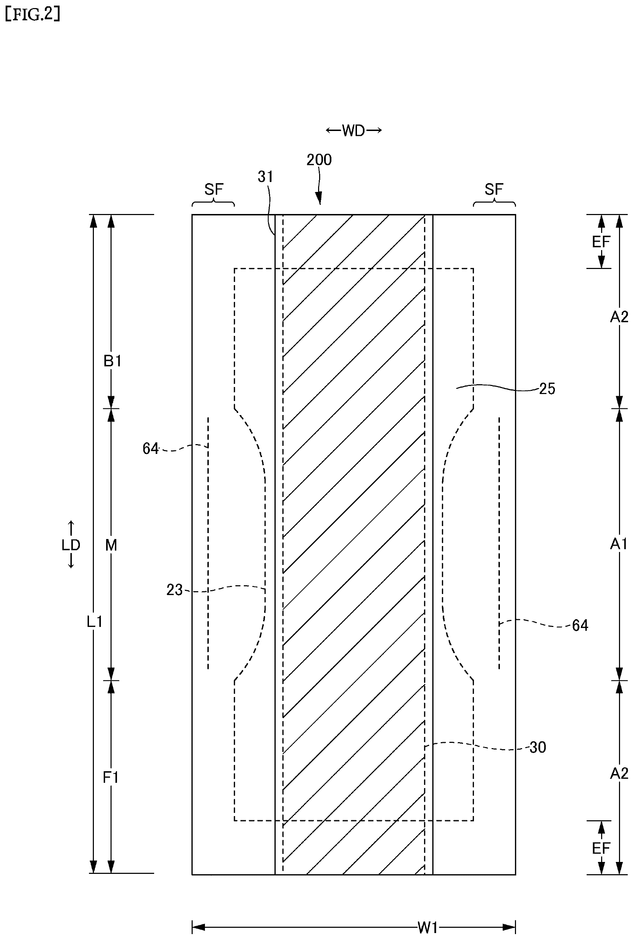

As shown in Figs. 2, 3, and 5, on the underside of the pad-type disposable diaper 200, i.e., on the underside of the exterior sheet 25 in the illustrated embodiment, a pressure-sensitive adhesive layer 30 is provided. Of the region having the pressure-sensitive adhesive layer 30, the zone located in the crotch section M is the first fixing zone A1, and the zones located forward and backward, respectively, of this first fixing zone A1 are the second fixing zones A2. It is preferred, as in the illustrated embodiment, that the pressure-sensitive adhesive layer 30 may be covered with a peelable release liner 31, which is peeled off upon use to expose the pressure-sensitive adhesive layer 30.

In the illustrated embodiment, the pressure-sensitive adhesive layer 30 is continuously disposed in the front-back direction LD so that the first fixing zone A1 and the second fixing zones A2 are arranged unintermittently, but the pressure-sensitive adhesive layer 30 may be disposed intermittently in the front-back direction LD so that the first fixing zone A1 and the second fixing zones A2 are spaced apart from each other in the front-back direction LD. The dimension in the front-back direction LD of the first fixing zone A1 may be 55 to 100% the dimension in the front-back direction LD of the crotch section M. The dimension in the front-back direction LD of the second fixing zone 2 located forward of the crotch section M may be 70 to 100% the dimension in the front-back direction LD of the front section F1, and the dimension in the front-back direction LD of the second fixing zone A2 located backward of the crotch section M may be 70 to 100% the dimension in the front-back direction LD of the back section B1.

The pressure-sensitive adhesive layer 30 and the marks 41, 42 to be discussed later preferably do not overlap, and the pressure-sensitive adhesive layer 30 preferably falls between the pair of right and left marks 41, 42 to be discussed later, but may partly overlap the marks 41, 42 to be discussed later.

The pressure-sensitive adhesive layer 30 is preferably disposed also in the end flaps EF, and preferably extends continuously from the front end over to the back end of the product as in the illustrated embodiment, but the overall pressure-sensitive adhesive layer 30 may be in such dimensions and arrangement as to fall within the region coincident with the absorber 23 in the thickness direction.

### <Marks>

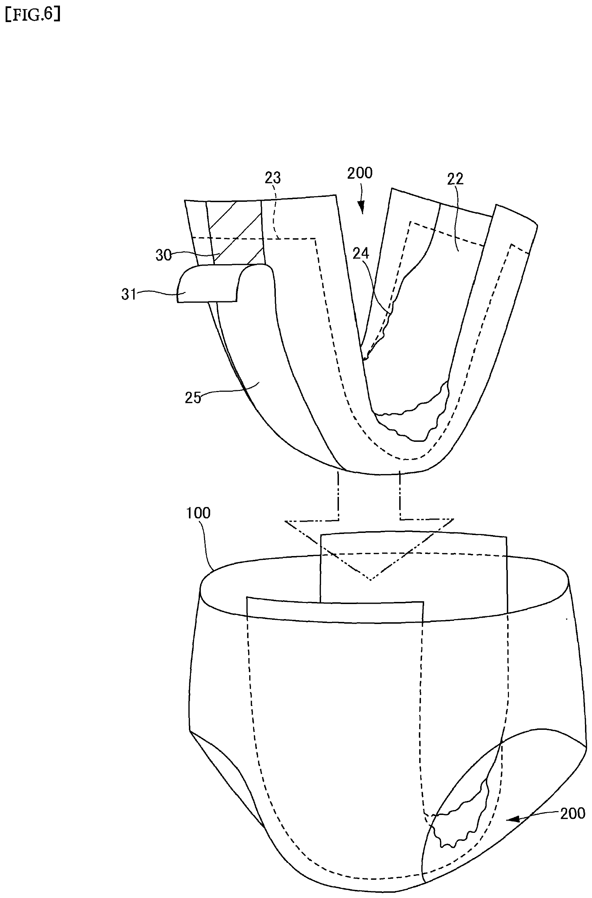

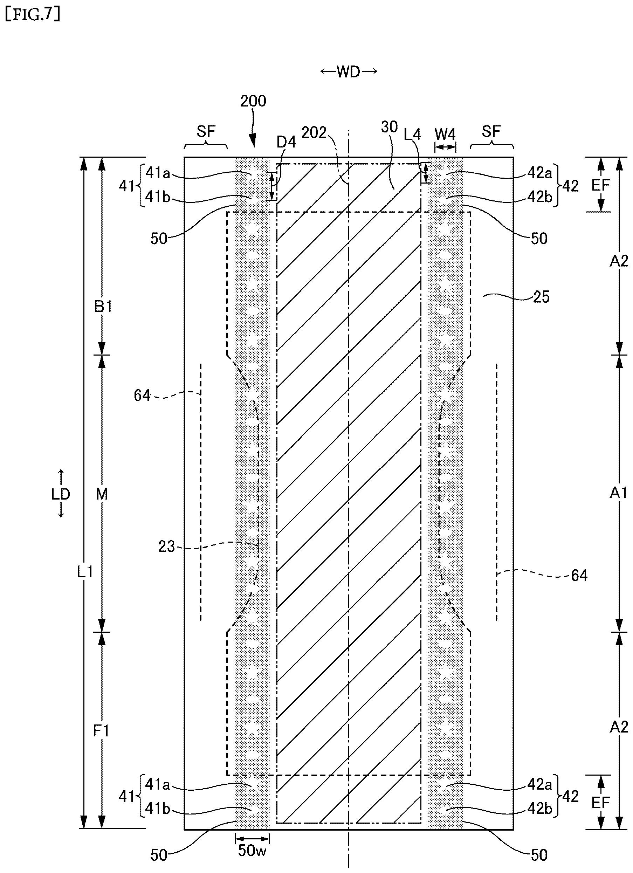

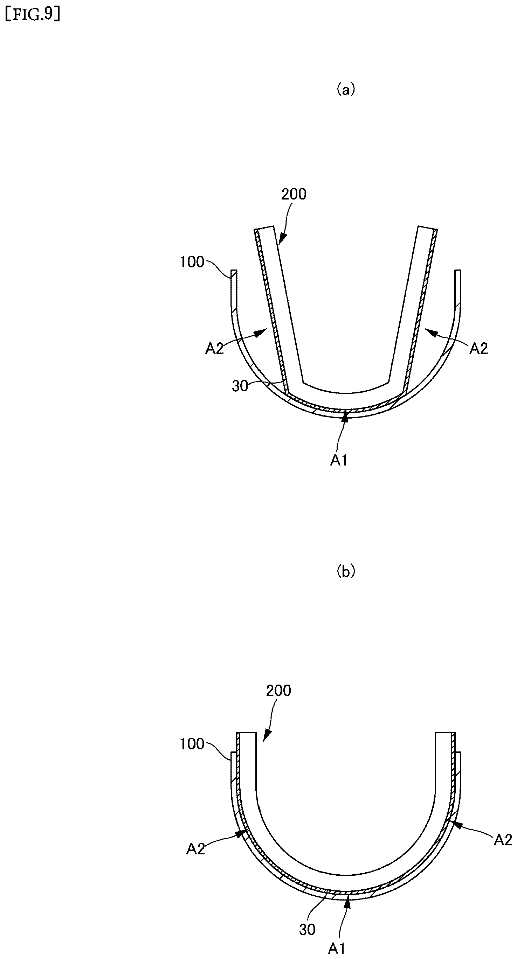

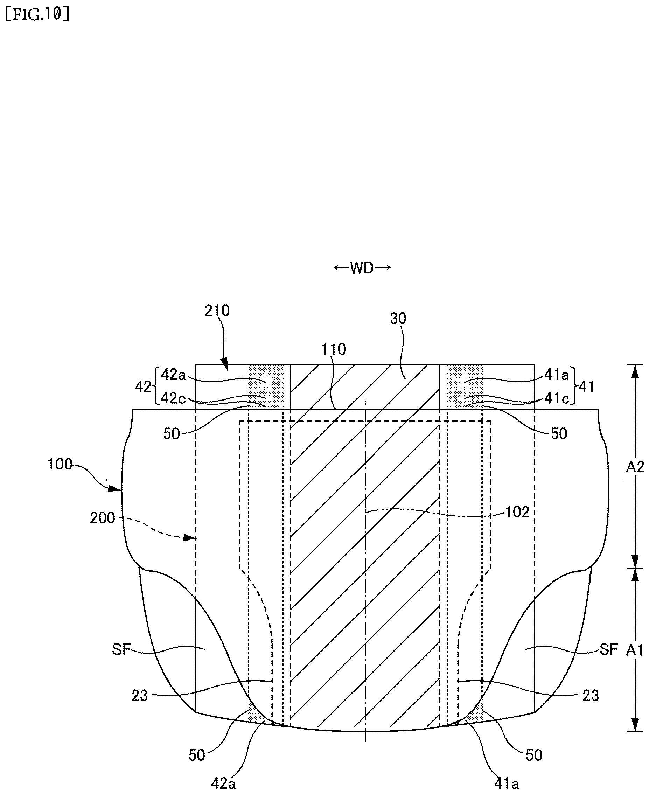

Characteristically, as shown in Fig. 7, in each of the front and back end portions of the product, a pair of right and left marks 41, 42 indicative of the positions in the front-back direction LD is provided so as to be visually observable from the underside of the product. The right and left marks 41, 42 in pair are spaced apart from each other in the width direction WD, and are arranged in symmetry with respect to the center line 102 passing the center of the width WD of the product (also referred to as bilateral symmetry). In attaching the present pad-type disposable diaper to underwear 100 as shown in Fig. 6, the crotch section M of the diaper (usually the middle of the diaper in the front-back direction) is aligned to the crotch section M of the underwear 100 as shown in Fig. 9, and the first fixing zone A1 located in the crotch section M of the diaper is fixed to the interior surface of the underwear 100 with the pressure-sensitive adhesive layer 30 provided on the underside of the first fixing zone A1, and then, using the marks 41, 42 provided in at least one of the front and back end portions of the diaper, positioning of the corresponding second fixing zone A2 may be checked. Specifically, as shown in Fig. 10, the second fixing zones A2 are placed on the interior surface of the underwear 100 so that at least one of the front and back end portions of the diaper sticks out of the waist opening edge 110 of the underwear 100. Then, the at least one sticking-out portion 210 is visually observed with respect to the underwear 100 and, when the number or the observed shape of the marks 41 seen on the right is different from the number or the observed shape of the marks 42 seen on the left unlike the state illustrated in Fig. 10, the corresponding second fixing zone A2 is found slanted. Further, even with the numbers of the marks 41, 42 observed on the right and the left being the same, when the center in the width direction WD of the area located between the right and left marks 41, 42 is displaced from the center line 102 passing the center of the width WD of the underwear 100, the second fixing zone A2 is found displaced in the width direction WD, though not slanted. Accordingly, in such cases, the position of the second fixing zone A2 is adjusted so that the marks 41, 42 in pair are observed in bilateral symmetry. In this positioning, the second fixing zone A2, if already adhered with its pressure-sensitive adhesive layer 30, is peeled off and repositioned before it is re-adhered. This repositioning may be repeated as necessary. In this way, by means of the marks 41, 42 provided in at least one of the end portions of the diaper, the corresponding second fixing zone A2 may be fixed in an appropriate position on the underwear 100 (state shown in Fig. 10) more easily.

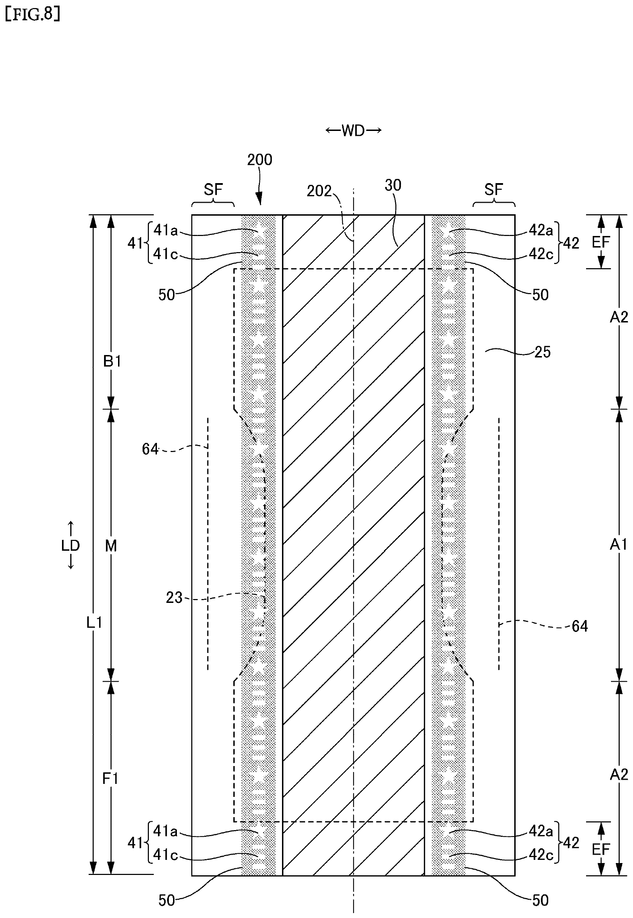

Each mark 41, 42 may be in any shape without limitation as far as the mark is indicative of the position in the front-back direction LD of the sticking-out portion 210 sticking out of the waist opening edge 110 of the underwear 100, and may suitably be selected from those indicative of the position in the shape of, such as circle, triangle, rectangle, star, or arrow, or such as characters or graphics. A plurality of the right and left marks 41, 42 may be provided on the respective right and left sides (provided that the numbers of the marks on the two sides are the same, as the marks should be arranged in bilateral symmetry). In the embodiment shown in Fig. 7, star marks 41a, 42a and elliptical marks 41b, 42b are arranged alternately and repeatedly at predetermined intervals in the front-back direction LD, whereas in the embodiment shown in Fig. 8, the marks 41, 42 are arranged at regular intervals in the front-back direction LD so that one star mark 41a, 42a is arranged between every three rounded laterally-rectangular marks 41c, 42c. In the illustrated embodiments, marks 41, 42 in pair are provided not only in the front and back end portions of the product but also between the front and back end portions. However, the marks 41, 42 in pair may not be provided in the portions other than the front and back end portions.

The marks 41, 42 may be provided by printing, or by profile processing, such as embossing, or by combination of these. The marks 41, 42 may be provided on any known member, or a member dedicated to this purpose may be added, as long as the marks 41, 42 are visually observable from the underside of the pad-type disposable diaper. For example, the marks 41, 42 may be printed on the under face of the liquid-impervious sheet 21 and rendered visually observable through the exterior sheet 25. Alternatively, a printed sheet made of, e.g., paper, on which the marks 41, 42 have been printed, may be interposed between the liquid-impervious sheet 21 and the exterior sheet 25 and rendered visually observable through the exterior sheet 25. Further, the marks 41, 42 may directly be printed on the under face of the exterior sheet 25.

The dimensions of each mark 41, 42 may suitably be decided and, in view of its purpose to indicate a local position and its visual observability in normal use, the dimension in the front-back direction LD (overall length) L4 is preferably 2 to 20 mm, and the dimension in the width direction WD (overall width) W4 is preferably 5 to 20 mm. When a plurality of the marks 41, 42 are repeatedly provided at intervals in the front-back direction LD, the center distance D4 between the marks adjacent in the front-back direction may suitably be decided, for example, to 2 to 15 mm. Note that the center distance D4 between the adjacent marks 41, 42 in the front-back direction LD may not be constant as long as it is regular. For example, when a plurality of types of marks 41, 42 are mixed as shown in Fig. 10, the center distance D4 in the front-back direction LD between the adjacent marks of different types may be different from the center distance D4 in the front-back direction LD between the adjacent marks of the same type.

In particular, as shown in the illustrated embodiments, when a plurality of the right and left marks 41, 42 in pair form scale marks arranged at regular intervals in the front-back direction LD on the respective right and left sides, the waist opening edge 110 of the underwear 100 may be checked for positioning with respect to the marks 41, 42 acting as the scale marks on the respective right and left sides, so that the appearance of the marks 41, 42 in pair with respect to the underwear 100 may preferably be visually observed for bilateral symmetry more easily.

Further, as shown in the illustrated embodiments, it is preferred that, in addition to the pairs of the right and left marks 41, 42, a pair of tape- or line-like longitudinal guide marks 50, 50 each linearly extending continuously along the front-back direction LD is provided at least in the sticking-out portions 210 sticking out of the waist opening of the underwear 100 (preferably over the entire length of the product), which allows intuitive grasp of the front-back direction LD of the product. In this case, as shown in the illustrated embodiments, it is particularly preferred that the marks 41, 42 indicative of the position in the front-back direction LD are arranged in the tape-like longitudinal guide marks 50, which allows visual observation of both of marks 50, 50 and marks 41, 42 at a glance and allows the marks 41, 42 indicative of the positions in the front-back direction LD to easily catch eyes.

In particular, the dimension 50w in the width direction of each longitudinal guide mark 50 may suitably be decided, and may be usually preferably about 7 to 30 mm.

For accurate positioning of the second fixing zones A2, it is preferred that the marks 41, 42 in pair are arranged spaced apart from each other with a larger distance in the width direction WD. However, with the pad-type disposable diaper 200 having the side flaps SF as shown in the illustrated embodiments, a pair of right and left marks 41, 42 provided on the side flaps, which are prone to deformation, may be hard to be visually checked for accurate symmetry due to, for example, the areas of the marks being flexed. On the other hand, as the region containing the absorber 23 is relatively higher in rigidity and thus less deformable, the marks 41, 42 in pair positioned corresponding to the respective side edge areas opposed in the width direction WD of the region containing the absorber 23 (in the areas overlapping the opposed side edge areas of the absorber 23, as well as the areas forward and backward thereof), as in the illustrated embodiments, may easily be visually checked for accurate symmetry of the positions of the right and left marks 41, 42, as the areas of the marks 41, 42 are not vulnerable to flexing.

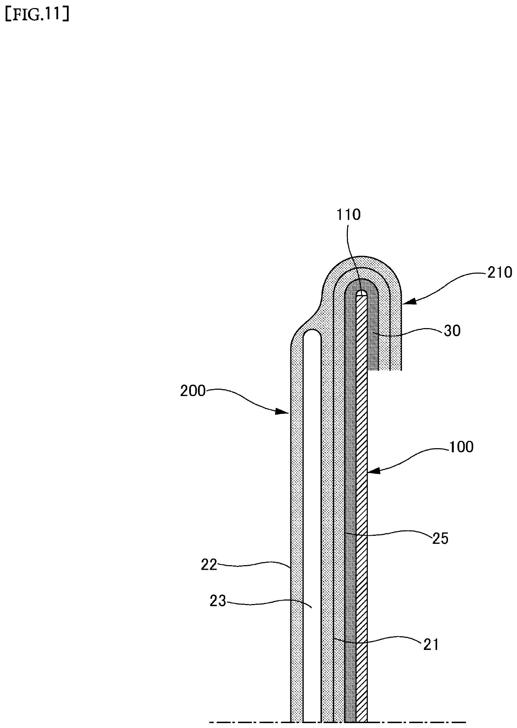

The sticking-out portion 210 of the diaper 200 sticking out of the waist opening edge 110 of the underwear 100, if left as it is, may deteriorate wearing comfort or air permeability, and thus may preferably be folded back toward the crotch section. However, the sticking-out portion 210 merely folded back may return to its initial state. In view of this, it is preferred that the at least one of the front and back end portions of the pad-type disposable diaper 200 is provided on its under face with the pressure-sensitive adhesive layer 30 as in the illustrated embodiments. In this way, the sticking-out portion 210 of the diaper sticking out of the waist opening edge 110 of the underwear 100 is not only folded back toward the crotch section, but this folded portion may also be fixed on the exterior surface of the underwear 100 with the pressure-sensitive adhesive layer 30, as shown in Fig. 11.

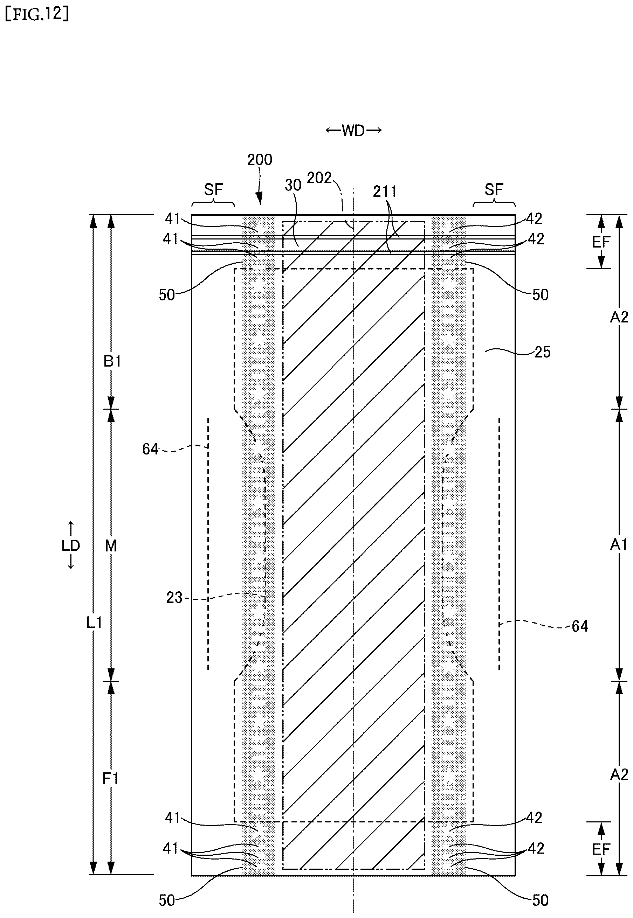

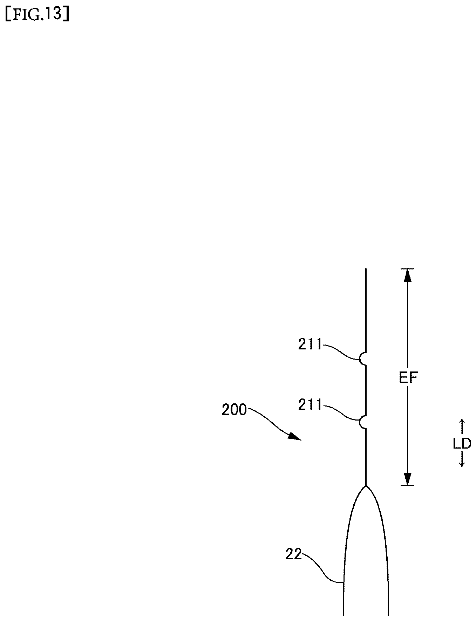

When the sticking-out portion 210 of the diaper sticking out of the waist opening edge 110 of the underwear 100 is to be folded back, the diaper is preferably provided with a plurality of easy-fold lines 211 for having the sticking-out portions folded back, arranged at intervals in the front-back direction LD and each extending in the width direction WD as shown in Figs. 12 and 13, which facilitates the folding process. The easy-fold lines 211 are provided through processing such that the folding back of the sticking-out portions is facilitated by their presence, and may be in the form of, for example, perforated lines or embossed lines having concaves formed in the under face of the target portions and convexes or concaves formed in the top face thereof. The easy-fold lines 211 may be provided in an end flap EF as in the illustrated embodiment, or in addition to or in place of this, may be provided in the region containing the absorber 23 (not shown).

### <Explanation of Terms in the Specification>

The following terms appearing in the present specification shall have the following means unless otherwise specified herein.
- The "front-back direction" refers to the direction shown by the reference sign LD (longitudinal direction) in the figures, whereas the "width direction" refers to the direction shown by the reference sign WD (right-left direction) in the figures, and the front-back direction and the width direction are orthogonal to each other.
- The "top side" refers to the side, when the article is worn, closer to the skin of the wearer, whereas the "underside" refers to the side, when the article is worn, away from the skin of the wearer.
- The "top face" refers to the face, when the article is worn, closer to the skin of the wearer, whereas the "under face" refers to the face, when the article is worn, away from the skin of the wearer.
- The "stretch rate" refers to a value with respect to the natural length being 100%. For example, a 200% stretch rate is synonymous with stretch in two folds.
- The "gel strength" is determined as follows. To 49.0 g of artificial urine (a mixture of 2 wt% urea, 0.8 wt% sodium chloride, 0.03 wt% calcium chloride dihydrate, 0.08 wt% magnesium sulfate heptahydrate, and 97.09 wt% ion-exchanged water), 1.0 g of superabsorber polymer is added and stirred with a stirrer. The resulting gel is left in a chamber with constant temperature and humidity at 40 °C at 60% RH for 3 hours, and then the temperature is returned to the ordinary temperature. The gel strength is measured in a curd meter (Curdmeter-MAX ME-500 manufactured by I. techno Engineering).
- The "basis weight" is determined as follows. A specimen or test piece is preliminarily dried, left in a laboratory or in apparatus under the standard conditions (23 ± 1 °C temperature and 50 ± 2% relative humidity in the testing location) until constant mass is attained. The preliminary drying refers to attaining constant mass from a specimen or test piece in the environment at a temperature of 100 °C. No preliminary drying may be performed on fibers with an official regain of 0.0%. From the test piece of the constant mass, a specimen of 100 mm × 100 mm size is cut out using a sampling template (100 mm × 100 mm). The weight of the specimen is measured and multiplied by 100 times to calculate the weight per 1 m², which is taken as the basis weight.
- The "thickness" is automatically measured using an automatic thickness meter (KES-G5 handy compression tester program) under a load of 0.098 N/cm² with the compression area of 2 cm².
- The "spread state" refers to the state in which an article is spread flatly without contraction (including any contraction, such as contraction by means of elastic members) or slack.
- The size of each part refers to the size not in the natural length state but in the spread state, unless otherwise specified.
- A test or measurement shall be, in the absence of description about environmental conditions, performed in a laboratory or in apparatus under the standard conditions (23 ± 1 °C temperature and 50 ± 2% relative humidity in the testing location).

### INDUSTRIAL APPLICABILITY

The present invention is applicable to pad-type disposable diapers having a pressure-sensitive adhesive layer on the under face thereof.

### DESCRIPTION OF REFERENCE SIGNS

- A1:: first fixing zone
- A2:: second fixing zone
- B1:: back section
- F1:: front section
- LD:: front-back direction
- M:: crotch section
- EF:: end flap
- SF:: side flap
- WD:: width direction
- 21:: liquid-impervious sheet
- 22:: top sheet
- 23:: absorber
- 23n:: narrowed portion
- 25:: exterior sheet
- 30:: pressure-sensitive adhesive layer
- 31:: release liner
- 41, 42:: mark
- 50:: longitudinal guide mark
- 60:: standup gather part
- 64:: side elastic member
- 100:: underwear
- 102:: center line
- 110:: edge of waist opening
- 200:: pad-type disposable diaper
- 210:: sticking-out portion
- 211:: easy-fold line

## Claims

1. A pad-type disposable diaper having a crotch section including a middle of the diaper in a front-back direction, a front section extending forward of the crotch section, and a back section extending backward of the crotch section,
the diaper having a first fixing zone located in the crotch section, and second fixing zones located forward of and backward of the first fixing zone,
the diaper comprising:
an absorber extending over the front section, the crotch section, and the back section, and
a pressure-sensitive adhesive layer provided on under faces of the first fixing zone and the second fixing zones,
wherein the diaper is to be fixed to underwear with at least one of front and back end portions thereof sticking out of an edge of a waist opening of the underwear,
wherein the at least one of the front and back end portions of the diaper is provided with a pair of right and left marks indicative of positions in the front-back direction so as to be visually observable from an underside of the diaper, and
wherein the marks in pair are spaced apart from each other in a width direction and are arranged in symmetry with respect to a center line passing a center of the width of the diaper.

2. The pad-type disposable diaper according to claim 1,
wherein each of the marks in pair comprises scale marks arranged at regular intervals in the front-back direction.

3. The pad-type disposable diaper according to claim 1 or 2,
wherein the diaper has a region containing the absorber, and side flaps exclusive of the absorber and extending laterally outwards beyond opposed side edges of the region containing the absorber, and
wherein the marks in pair are positioned in the end portion corresponding to respective side edge areas opposed in the width direction of the region containing the absorber.

4. The pad-type disposable diaper according to any one of claims 1 to 3,
wherein the at least one of the front and back end portions has on an under face thereof a pressure-sensitive adhesive layer.

5. The pad-type disposable diaper according to any one of claims 1 to 4, further comprising a plurality of easy-fold lines arranged at intervals in the front-back direction and each extending in the width direction, for having the at least one of the front and back end portions folded back toward the underside.
